# EUROPEAN PATENT APPLICATION

(11) **EP 4 742 258 A1**
(43) Date of publication of application: **13.05.2026**
(21) Application number: 24836093.5
(22) Date of filing: 04.07.2024
(51) Int. Cl.: G16H 20/00

(54) **COGNITIVE BEHAVIORAL THERAPY SUPPORT METHOD, COGNITIVE BEHAVIORAL THERAPY SUPPORT DEVICE, COGNITIVE BEHAVIORAL THERAPY SUPPORT PROGRAM, AND RECORDING MEDIUM**

(30) Priority: 04.07.2023 JP 2023109974
(71) Applicant: Hedgehog Medtech, Inc., Tokyo 112-0004 (JP)
(72) Inventor: KAWATA Yumi, Tokyo 112-0004 (JP); ISHIZAKA Hiroaki, Tokyo 112-0004 (JP)
(74) Representative: Aera A/S
(86) International application number: PCT/JP2024/024166
(87) International publication number: WO 2025/009581

(57) **Abstract**

The present disclosure relates to providing cognitive behavioral therapy appropriately while reducing difficulties and distress associated with keeping record of a patient's condition (including, for example, the time and effort required to recall the situation in which symptoms occurred, the possibility of remembering such situations only vaguely or with distorted memory, and the like). The present disclosure provides a computer-implemented method for supporting cognitive behavioral therapy, comprising: keeping record of a symptom or condition of a patient and at least one item of categorical information selected from a trigger, presence/absence and/or detail of medication, a prodromal symptom, date and time, a treatment, influence, and a location for a certain period; generating, based on the record of the symptom or condition and the categorical information, information for recalling a past situation in which the symptom occurred, and providing the patient with the information; providing the patient with information for learning about the symptom or condition of the patient; and providing the patient with information that promotes cognitive behavioral therapy.

## Description

### [Technical Field]

The present invention relates to support methods for cognitive behavioral therapy (CBT) for the treatment of diseases or symptoms such as migraine and premenstrual syndrome (PMS), support programs for cognitive behavioral therapy, support systems for cognitive behavioral therapy, and recording media.

### [Background Art]

In recent years, migraine has become a significant social problem. In Japan, the estimated number of migraine patients is 8.4 million, with a prevalence rate of 8.4% (Non-Patent Literature 1).

Premenstrual syndrome (PMS) refers to various physical and psychological symptoms that women experience prior to menstruation. Approximately 20% to 40% of all women experience some form of PMS symptoms, and the prevalence of severe PMS (premenstrual dysphoric disorder) is said to be about 3% to 8% (Non-Patent Literature 2).

These diseases or symptoms not only interfere with daily life but also exert a significant social effect because many patients are of working age.

For migraine, symptomatic treatment using analgesics is currently mainstream; however, the use of analgesics entails problems such as dependence from long-term use, gastrointestinal disorders, drug overuse, and drug interactions. Furthermore, when the cause of headaches lies in lifestyle habits such as stress, fatigue, or irregular lifestyle, analgesics may provide temporary relief but cannot resolve the underlying cause.

For PMS, for example, analgesics are used for pain such as abdominal pain or headaches; diuretics are used for swelling (edema); and anxiolytics are used for emotional instability. For moderate or more severe cases, antidepressants and other medications are used depending on the symptoms such as depression.

On the other hand, cognitive behavioral therapy has been proven effective in many prior studies and actual clinical practice, but the number of physicians able to provide it has been limited in terms of effort and expertise required. Recently, however, it has become possible to provide simple and highly effective therapy through application programs on information terminals and other means.

For example, Patent Literature 1 discloses a system for treating, preventing, or doing self-care for menstrual-related symptoms, the system comprising at least: a memory apparatus that stores a symptom memory which is a record including information related to the user's menstrual cycle and a plurality of items used to diagnose menstrual-related symptoms and a treatment module based on psychotherapy for treating, preventing, or self-caring for menstrual-related symptoms; and a user terminal operated by the user, wherein the system provides the patient with a treatment module, at a predetermined timing in the menstrual cycle, based on psychotherapy for treatment, prevention, or self-care corresponding to the user's menstrual-related symptoms, from the items of menstrual-related symptoms stored in the symptom memory of the user and the data stored in the system showing the correspondence between menstrual-related symptoms and treatment modules.

Patent Literature 2 discloses a system comprising a server functioning as a control device and an information processing terminal used by the patient, wherein the system has:
a block having a function of assessing to what extent the patient behaves adaptively or maladaptively regarding psychosocial problems, life adaptation, stress, etc.; and/or
a block having a function of assessing to what extent the patient behaves adaptively or maladaptively for symptoms characteristic of a specific disease;
and wherein the system has a function of analyzing the assessment results of the block, classifying the patients into a plurality of specific groups based on behavior using a combination of statistical processing of multiple indices including mental disorder indicators, and thereby classifying behavioral characteristics related to psychogenic diseases.

Patent Literature 3 discloses a mental healthcare support device equipped with contradiction determination means that determines whether there is inconsistency between automatic thought and feeling, based on an automatic thought category made by categorizing an automatic thought expression representing the client's automatic thought or the client's automatic thought into a predetermined category and a feeling category made by categorizing the feeling expression representing the client's feelings when the automatic thought arose or the client's feelings into a predetermined category.

Patent Literature 4 discloses that cognitive behavioral therapy includes the "column method" and "behavior and (accompanying) mood diary," in which a user inputs actions that improved their mood and the mood at that time to accumulate data on what actions improve their mood, and the therapy proceeds by analyzing behavioral patterns.

### [Prior Art References]

### [Patent Literature]

[Patent Literature 1] Japanese Patent No. 7138839
[Patent Literature 2] Japanese Unexamined Patent Publication No. 2022-068362
[Patent Literature 3] Japanese Re-publication No. 2014/045546
[Patent Literature 4] Japanese Unexamined Patent Publication No. 2019-185586

### [Non-Patent Literature]

[Non-Patent Literature 1] Sakai F, Igarashi H. *Cephalalgia* 1997; 17: 15-22
[Non-Patent Literature 2] Takeda et al., *Archives of Women's Mental Health*, July 2006; 9(4): 209-12

### [Summary of the Invention]

### [Problems to be Solved by the Invention]

In migraine patients, "excessively negative thinking" about migraine leads to feelings of anxiety, depression, or anger and in some cases the migraine becomes worse due to stress caused by the feelings or in other cases the feelings result in inappropriate behaviors toward pain (e.g., excessive use of analgesics).

For such migraine patients, cognitive behavioral therapy (CBT), also referred to as "CBT intervention," is effective. For example, there are surveys showing that CBT intervention improved headaches by 46%, and reports indicating a 65% reduction in migraine-related disability (MIDAS score) (Japan Headache Society Clinical Improvement Committee, *Migraine Cognitive Behavioral Therapy Manual (For Therapists), First Edition, 2021, pp. 10-14).

Cognitive restructuring is a kind of CBT, by which patients aim to objectively recognize their own excessively negative and biased thoughts, and to newly acquire more balanced and flexible ways of thinking.

In cognitive restructuring, the process of having the user (patient) retrospect on what actions they were taking and what thoughts and emotions they were experiencing when symptoms occurred is important. For this reason, a sheet called "column method" is generally used, in which the user is made to describe their symptoms and the thoughts and behaviors at that time.

However, it is generally difficult to recall one's conditions and emotions at the onset of headaches and the like, and in some cases it may even exacerbate distress. That is, recalling the episode may take time and effort; the situation may be remembered only vaguely or with distorted memory; or recalling the episode may itself cause additional headache or stress.

However, conventional technologies, including the above-mentioned Patent Literatures, have not attempted to provide cognitive restructuring while alleviating such difficulties and distress accompanying retrospection (including the time and effort required to recall the episode, the possibility of vague or distorted memory of the episode, etc.).

The present invention has been made in view of such issues, and aims to provide the patient with information for recalling past situations in which symptoms occurred, while alleviating difficulties and distress accompanying retrospection in the patient's conditions (including the time and effort required to recall the episode, the possibility of vague or distorted memory of the episode, etc.), and to provide CBT appropriately. The method disclosed herein is not limited to migraines but can also be applied to other symptoms or conditions such as PMS.

### [Means for Solving the Problems]

The method of the present invention is a computer-implemented method for supporting cognitive behavioral therapy, including keeping record of a symptom or condition of a patient and at least one item of categorical information selected from a trigger, medication, a prodromal symptom, date and time, a treatment, influence, and a location for a certain period; generating information for recalling a past situation in which the symptom occurred, based on the record of the symptom or condition and the categorical information, and providing the patient with the information; providing the patient with information for learning about the symptom or condition of the patient; and providing the patient with information that promotes the cognitive behavioral therapy.

More specifically, the present disclosure encompasses the following aspects:
[Aspect 1] A computer-implemented method for supporting cognitive behavioral therapy, the method comprising:
   keeping record of a symptom or condition of a patient and at least one item of categorical information selected from a trigger, presence/absence and/or detail of medication, a prodromal symptom, date and time, a treatment, influence, and a location for a certain period;
   generating and providing the patient with information for recalling a past situation in which the symptom occurred, based on the record of the symptom or condition and the categorical information;
   providing the patient with information for learning about the symptom or condition of the patient; and
   providing the patient with information that promotes the cognitive behavioral therapy.
[Aspect 2] The method according to Aspect 1, further comprising keeping record of the symptom or condition and the categorical information by selecting one item of information from options thereof.
[Aspect 3] The method according to Aspect 2, wherein the options in the record of the symptom or condition and the categorical information are provided with one of the options preselected.
[Aspect 4] The method according to Aspect 3, wherein the selection of the preselected option in the record of the symptom or condition and the categorical information is made, based on a record of past information of the patient.
[Aspect 5] The method for supporting cognitive behavioral therapy according to any one of Aspects 1 to 4, further comprising:
   keeping record a symptom or condition of a patient by text input into a text field for a certain period; and
   displaying a text candidate predicted from a few input letters; the text candidate being predicted based on past records of information of the patient.
[Aspect 6] The method for supporting cognitive behavioral therapy according to any one of Aspects 1 to 5, further comprising:
   performing statistical processing and/or clustering processing on data in a database made by keeping record of symptoms or conditions of multiple patients and at least one item of categorical information selected from a trigger, presence/absence and/or detail of medication, a prodromal symptom, date and time, a treatment, influence, and a location for a certain period to create multiple patient classifications; and
   identifying a patient classification to which a patient belongs, based on a symptom or condition of the patient recorded for a certain period and the categorical information selected from a trigger, presence/absence and/or detail of medication, a prodromal symptom, date and time, a treatment, influence, and a location.
[Aspect 7] The method for supporting cognitive behavioral therapy according to any one of Aspects 1 to 6, further comprising:
   presenting, to the patient, information on the relationship between a symptom or condition of a patient recorded for a certain period and frequency of occurrence of at least one item of information selected from a trigger, presence/absence and/or detail of medication, effects of medication, a prodromal symptom, date and time, a treatment, influence, and a location.
[Aspect 8] The method for supporting cognitive behavioral therapy according to any one of Aspects 1 to 7, further comprising displaying a symptom or condition of a patient recorded for a certain period and frequency of occurrence of at least one item of information selected from a trigger, presence/absence and/or detail of medication, effects of medication, a prodromal symptom, date and time, a treatment, influence, and a location in a ranked order.
[Aspect 9] The method for supporting cognitive behavioral therapy according to any one of Aspects 1 to 8, further comprising providing the patient with information promoting cognitive restructuring based on the symptom or condition and the categorical information and/or a video explaining content of cognitive restructuring.
[Aspect 10] The method for supporting cognitive behavioral therapy according to any one of Aspects 1 to 9, further comprising providing the patient with information promoting behavioral activation based on the symptom or condition and the categorical information and/or a video explaining content of behavioral activation.
[Aspect 11] The method for supporting cognitive behavioral therapy according to any one of Aspects 1 to 10, further comprising providing the patient with information promoting relaxation based on the symptom or condition and the categorical information and/or a video explaining content of relaxation.
[Aspect 12] The method for supporting cognitive behavioral therapy according to any one of Aspects 1 to 11, further comprising providing the patient with information promoting biofeedback based on the symptom or condition and the categorical information and/or a video explaining content of biofeedback.
[Aspect 13] The method for supporting cognitive behavioral therapy according to any one of Aspects 1 to 12, further comprising:
   keeping record of the patient's condition after the implementation of cognitive behavioral therapy; and
   comparing the patient's condition before and after the implementation of cognitive behavioral therapy.
[Aspect 14] The method for supporting cognitive behavioral therapy according to any one of Aspects 1 to 13, further comprising:
   registering multiple dates/times scheduled for training of cognitive behavioral therapy for a certain period;
   providing an explanatory video when the patient performs the training;
   keeping record of whether the training was performed at each scheduled date and time;
   when the training was conducted, keeping record of the patient's impressions after the training; and
   presenting, to the patient, relationship between the symptom or condition of the patient and the training implementation status and impressions recorded for a certain period.
[Aspect 15] The method for supporting cognitive behavioral therapy according to Aspect 14, further comprising prompting the patient to perform the training a predetermined period before the scheduled training date and time.
[Aspect 16] A cognitive behavioral therapy support apparatus comprising:
   a patient state recording means for keeping record of a symptom or condition and at least one item of categorical information selected from a trigger, presence/absence and/or detail of medication, a prodromal symptom, date and time, a treatment, influence, and a location for a patient for a certain period;
   a recall information providing means for generating, based on the record of the symptom or condition and the categorical information, information for recalling a past situation in which the symptom occurred, and providing the patient with the information;
   a learning information providing means for providing the patient with information for learning about the symptom or condition of the patient; and
   a cognitive behavioral therapy information providing means for providing the patient with information promoting cognitive behavioral therapy.
[Aspect 17] A program for causing a computer to function as each means of the cognitive behavioral therapy support apparatus according to Aspect 16.
[Aspect 18] A computer-readable recording medium storing a program for causing a computer to function as each means of the cognitive behavioral therapy support apparatus according to Aspect 16.
[Aspect 19] The method for supporting cognitive behavioral therapy according to any one of Aspects 1 to 14, wherein the symptom or condition is migraine.
[Aspect 20] The method for supporting cognitive behavioral therapy according to Aspect 19, wherein the patient is a patient who has shown insufficient response to treatment with an anti-CGRP antibody or an anti-CGRP receptor antibody.
[Aspect 21] The method for supporting cognitive behavioral therapy according to any one of Aspects 1 to 14, wherein the symptom or condition is PMS.
[Aspect 22] The method for supporting cognitive behavioral therapy according to any one of Aspects 1 to 14, further comprising prompting the patient so that the number of days on which the record is kept accounts for 40% or more of the days in a certain period.
[Aspect 23] The method for supporting cognitive behavioral therapy according to any one of Aspects 1 to 14, wherein the symptom or condition is selected from the group consisting of a headache, a tension-type headache, a medication overuse headache, a hormonal headache, chronic low back pain, chronic pain syndrome (CRPS), fibromyalgia, joint pain, PMDD (premenstrual dysphoric disorder), menstrual-related disorder, depression, anxiety disorder, panic disorder, social anxiety disorder, post-traumatic stress disorder, obsessive-compulsive disorder, insomnia, eating disorder, schizophrenia, menopausal disorders, irritable bowel syndrome, hyperhidrosis, atopic dermatitis, substance abuse, and mental disorder.
[Aspect 24] The method for supporting cognitive behavioral therapy according to any one of Aspects 1 to 14, wherein the information for recalling a past situation in which a symptom occurred includes at least one selected from the group consisting of headache symptom, a prodromal symptom, a concomitant symptom, a trigger, a location at the time of an event, level of physical fatigue, level of mental fatigue, and sleep duration.
[Aspect 25] The method for supporting cognitive behavioral therapy according to Aspect 24, wherein the information for recalling a past situation in which a symptom occurred comprises at least a prodromal symptom and a concomitant symptoms.

### [Effects of the Invention]

According to the present invention, it becomes possible to appropriately provide cognitive behavioral therapy while alleviating the difficulties and distress accompanying the recording of a patient's conditions (including the time and effort required to recall the episode, the possibility of vague or distorted memory of the episode, etc.).

### [Brief Description of the Drawings]

[FIG. 1] FIG. 1 is a schematic configuration diagram of a computer capable of implementing the cognitive behavioral therapy support method according to an aspect of the present invention.
[FIG. 2] FIG. 2 is a screen transition diagram schematically showing transitions between main screens of a smartphone application program according to an aspect of the present invention.
[FIG. 3] FIG. 3 is a screen transition diagram showing the screen transitions in the "Home" screen.
[FIG. 4] FIG. 4 is a diagram showing the "Diary Record" screen.
[FIG. 5] FIG. 5 is a flowchart showing the processing flow for creating patient classifications by clustering data in a database for multiple patients.
[FIG. 6] FIG. 6 is a graph showing, for patients (n=52) who have migraine with at least two attacks per month or more than four days of attacks, the changes from baseline in the number of migraine days per four weeks at weeks 4, 8, 12, 16, 20, and 24 in the treatment with a headache treatment application according to this disclosure for 24 weeks.
[FIG. 7] FIG. 7 is a graph showing the effect of the headache treatment application according to this disclosure for patients (n=12) with migraine with at least two attacks per month or more than four days of attacks who have shown insufficient response to treatment with an anti-CGRP antibody or anti-CGRP receptor antibody.

### [Embodiments for Carrying Out the Invention]

Hereinafter, the cognitive behavioral therapy support method according to embodiments of the present invention will be described with reference to the drawings as appropriate. Cognitive behavioral therapy (CBT) is an effective approach for treating physical pain and chronic pain affected by psychological factors. Examples of conditions for which CBT is applicable include:
1. Chronic Pain Syndrome (CRPS): CBT is effective for complex pain syndromes such as chronic pain syndrome. Patients learn cognitive and behavioral strategies to address psychological factors such as fear, anxiety, and stress related to pain and improve quality of life.
2. Fibromyalgia: CBT is also effective for managing chronic symptoms related to pain and fatigue such as fibromyalgia. By changing cognitive patterns and behavioral patterns toward pain, reduction of symptom and improvement of quality of life are promoted.
3. Chronic Low Back Pain: CBT is also effective for treating chronic low back pain and other chronic back or joint pains. Patients acquire practical skills to address psychological factors associated with pain, such as stress and anxiety, and alleviate symptoms.
4. Headache: CBT is also useful in managing chronic headaches such as migraine and tension-type headache. Patients recognize triggers and cognitive patterns associated with headaches, and learn stress management and relaxation techniques to alleviate symptoms.
5. Joint Pain: CBT is also effective for joint pain and arthritis. Patients aim to alleviate symptoms and improve daily life by changing cognitive patterns toward pain and increasing physical activity.
6. Menopausal Disorders: CBT is also effective for menopausal disorders. Patients recognize cognitive patterns related to drug abuse and learn to address anxiety and stress and acquire practical skills to alleviate symptoms.
7. Hyperhidrosis: CBT is also effective for hyperhidrosis. Patients learn to address psychological factors such as stress and anxiety related to the symptoms and acquire practical skills to alleviate symptoms.
8. Atopic Dermatitis: CBT is also effective for atopic dermatitis. Patients address stress and anxiety related to symptom exacerbation and acquire practical skills to alleviate symptoms.
9. Substance Abuse: CBT is also effective for drug abuse. Patients recognize cognitive patterns related to substance abuse, and learn to address anxiety and stress and acquire practical skills to reduce symptoms.

In these pain-related conditions, CBT changes pain perception and stress responses and serve as an effective treatment method to support symptom relief and quality-of-life improvement. Moreover, CBT has been demonstrated to be effective for many mental disorders, such as depression, anxiety disorders (panic disorder, social anxiety disorder, post-traumatic stress disorder, obsessive-compulsive disorder, etc.), insomnia, eating disorders, and schizophrenia, and is now widely used.

### (First Embodiment)

The cognitive behavioral therapy support method according to the first embodiment of the present disclosure is a computer-implemented method for supporting cognitive behavioral therapy, comprising: keeping record of a symptom or condition of a patient, which may include the patient's menstrual cycle,and at least one item of categorical information selected from a trigger, presence/absence and/or detail of medication, a prodromal symptom (also referred to as a premonitory symptom), date and time, a treatment, influence, and a location, weather information, and treatment actually practiced, for a certain period; generating, based on the record of the symptom or condition and the categorical information, information for recalling past situation in which the symptom occurred, and providing the patient with the information; providing the patient with information for learning about their own symptom or condition; and providing the patient with information that promotes cognitive behavioral therapy.

Here, the term "categorical" means "belonging to a category/classification" or "classified."

The term "influence" refers to the influence of the symptom or condition of the patient, such as "I was unable to work due to headache for several days." "Influence" can include, for example, influence on work or school, housework, family events, socializing, errands, and leisure activities.

The "symptom or condition of a patient" can include, for example, pain severity, location of pain, onset pattern, headache symptoms, concomitant symptoms, level of physical fatigue, level of mental fatigue, and sleep duration.

The "symptom or condition" can include, for example, headaches such as migraines, tension-type headaches, medication overuse headaches and hormonal headaches, chronic low back pain, chronic pain syndrome (CRPS), fibromyalgia, joint pain, menstrual-related disorders such as PMS (premenstrual syndrome) and PMDD (premenstrual dysphoric disorder), and mental disorders such as depression; anxiety disorder (panic disorder, social anxiety disorder, post-traumatic stress disorder, obsessive-compulsive disorder, etc.); insomnia, eating disorder, and schizophrenia.

The "patient" may include, for example, migraine patients for whom treatment with an anti-CGRP antibody or anti-CGRP receptor antibody is ineffective or insufficient.

The "a certain period" may be, for example, 1 week or longer, 2 weeks or longer, 3 weeks or longer, 4 weeks or longer, 8 weeks or longer, 12 weeks or longer, 16 weeks or longer, 20 weeks or longer, 24 weeks or longer, 28 weeks or longer, 32 weeks or longer, or 36 weeks or longer. The "a certain period" may also be, for example, within 40 weeks, within 36 weeks, within 32 weeks, within 28 weeks, within 24 weeks, within 20 weeks, within 16 weeks, within 12 weeks, within 8 weeks, within 4 weeks, within 3 weeks, or within 2 weeks. The "a certain period" may further be, for example, 1 week, 2 weeks, 3 weeks, 4 weeks, 8 weeks, 12 weeks, 16 weeks, 20 weeks, 24 weeks, 28 weeks, 32 weeks, 36 weeks, or 40 weeks.

During the treatment, it is desirable that the patient keep record of their own information on as many days as possible, ideally every day. The computer-implemented cognitive behavioral therapy support method according to this disclosure may further include prompting the patient so that the number of days on which the record is kept accounts for a sufficient percentage within the certain period of time, for example, 25% or more, 30% or more, 40% or more, 50% or more, 60% or more, 70% or more, 80% or more, 90% or more, 95% or more, or 100%.

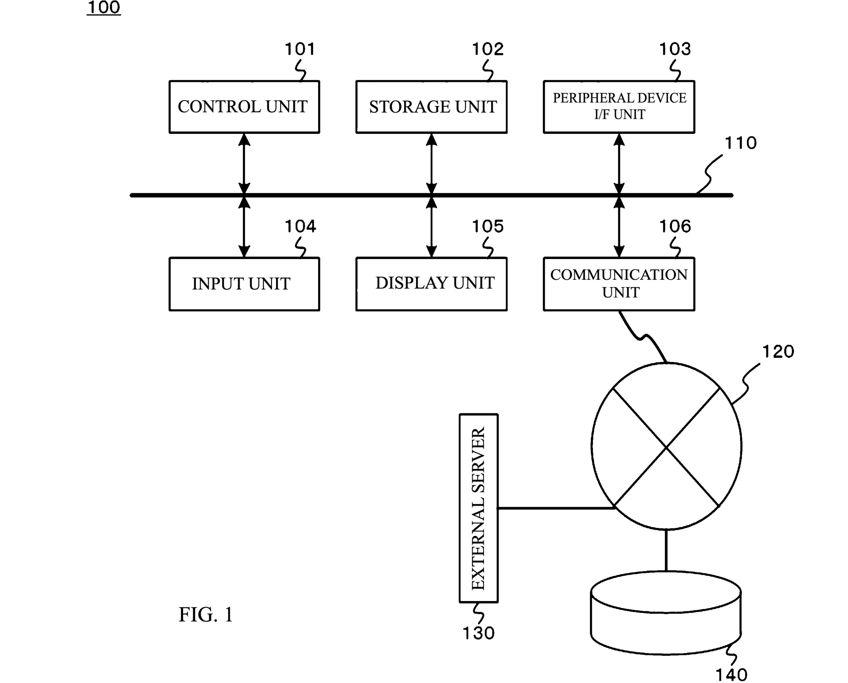

FIG. 1 shows the schematic configuration of a computer capable of implementing the cognitive behavioral therapy support method according to the first embodiment of the present invention.

In FIG. 1, reference numeral 100 denotes a computer, which includes a control unit 101, a storage unit 102, a peripheral device interface (I/F) unit 103, an input unit 104, a display unit 105, and a communication unit 106, all connected via a bus 110.This configuration is illustrative, and various other configurations may be adopted as appropriate.

The control unit 101 is composed of a CPU (Central Processing Unit), ROM (Read Only Memory), RAM (Random Access Memory), etc. The CPU loads programs stored in the storage unit 102, ROM, or recording media into the work memory area of the RAM and executes them to control the operation of the devices connected via the bus 110, thereby implementing the processing performed by the computer. The ROM is a non-volatile memory that stores programs such as the computer 100's boot program or BIOS, as well as data. The RAM is a volatile memory that temporarily stores programs and data loaded from the storage unit 102, ROM, or recording media, and also is provided with a work area that the control unit 101 uses when doing various processing tasks. The storage unit 102 is, for example, a hard disk drive (HDD) and stores programs executed by the control unit 101 and various other data.

The peripheral device I/F unit 103 is a port for connecting the computer 100 to peripheral devices. It may be composed of USB, IEEE1394, RS-232C, and the like. The connection may be wired or wireless. The input unit 104 includes input devices such as a keyboard, a pointing device (e.g., mouse), numeric keypad, etc., allowing the user to input operation commands, action instructions, or data into the computer 100. The display unit 105 is a logical circuit or device driver for displaying images, graphics, and other visuals on a display device such as an LCD panel. The input unit 104 and the display unit 105 may be integrally configured as a touch display. The communication unit 106 includes a communication control device and a communication port, and serves as a wired or wireless communication interface with a network 120. The bus 110 is a communication pathway mediating the exchange of control signals, data signals, and the like between each of the units.

The network 120 can also be connected to an external server 130 and a database 140. The computer 100 may be formed as an integrated computer system, functionally connecting to these at any time.

The computer 100 capable of implementing the method of the present invention may take various forms, including smartphones, tablet PCs, desktop PCs, servers, and mainframes. Even devices not typically referred to as computers, such as portable music players or digital cameras, may serve as a computer implementing the present invention if equipped with the above configuration. However, the method of the present invention is typically implemented as an application program running on a smartphone. Accordingly, in the following description, the display unit 105 is assumed to be a touch display integrated with the input unit 104. Unless otherwise specified, the terms "button," "slider," and other interface elements refer to graphical user interface (GUI) components displayed on the display unit 105 for receiving user input.

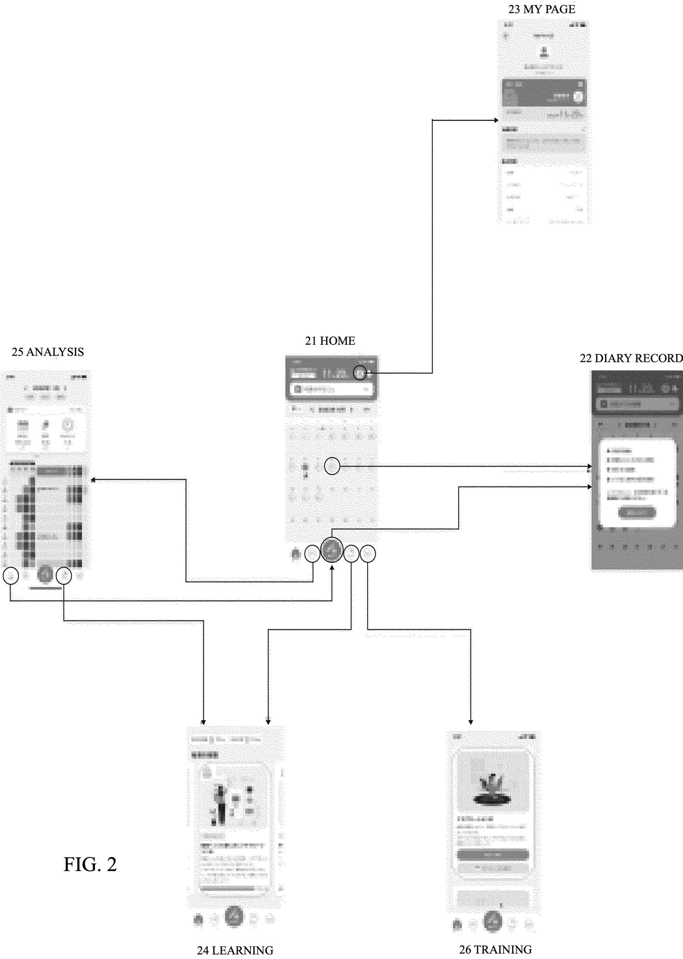

FIG. 2 is a screen transition diagram schematically showing transitions between main screens of a smartphone application program according to an embodiment of the method of the present invention. Here, headache is exemplified as the symptom.

The application program according to this embodiment is launched, when the user issues a start instruction (by selecting the application icon on the OS screen), or when a notification is sent to the user at the preset time (e.g., 8:00 a.m. or 8:00 p.m.) and the notification message is selected.

In FIG. 2, reference numeral 21 denotes the "Home" screen, 22 denotes the "Diary Record" screen, 23 denotes the "My Page" screen, 24 denotes the "Learn" screen, 25 denotes the "Analysis" screen, and 26 denotes the "Training" screen.

The "Home" screen 21 is the main screen normally displayed when the user launches the application program, and it provides the user with access to various functions and contents of the application program. On the very first launch, however, an "Initial Interview" screen (not shown) is displayed, receiving the input of information such as employment type and smoking habits.

The "Diary Record" screen 22 is a screen on which the user records symptoms, triggers, medication use, prodromal symptom, etc., by selecting from options, with the entries associated with a specific date.

The "My Page" screen 23 is a screen that displays the user's basic information such as name and date of birth, and interview information such as occupation and lifestyle habits.

The "Learn" screen 24 is a screen that displays learning content such as instructions on how to use the application program, knowledge regarding headaches, and explanations of cognitive behavioral therapy.

The "Analysis" screen 25 is a screen that displays data such as occurrence patterns and duration of headache.

The "Training" screen 26 is a screen that provides the user with training related to cognitive behavioral therapy.

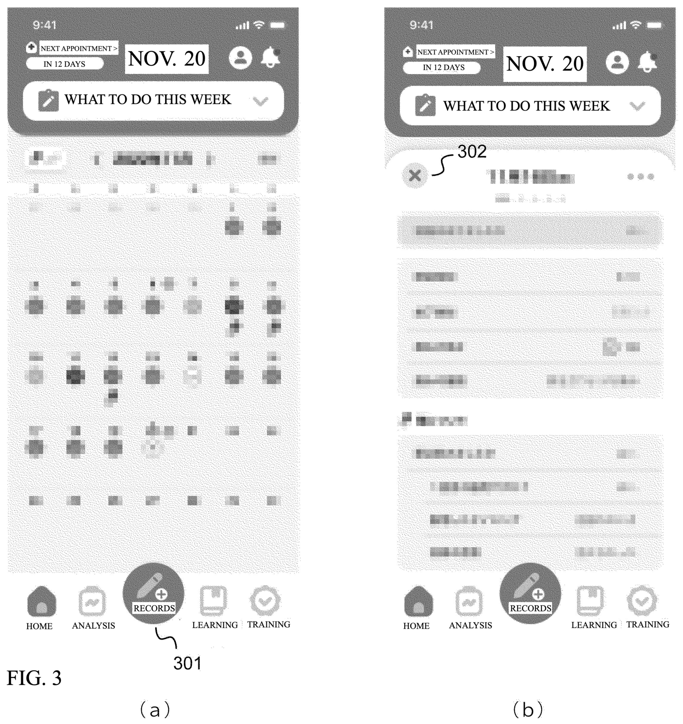

FIG. 3 is a screen transition diagram showing the transitions on the "Home" screen 21.

When the application program according to the present embodiment is launched by the user (on the second and subsequent launches), the screen shown in FIG. 3(a) is displayed on the display unit 105.

In FIG. 3(a), when the user selects the "Record" button 301, the display transitions to FIG. 3(b). FIG. 3(b) is a screen for performing the "Diary Record".

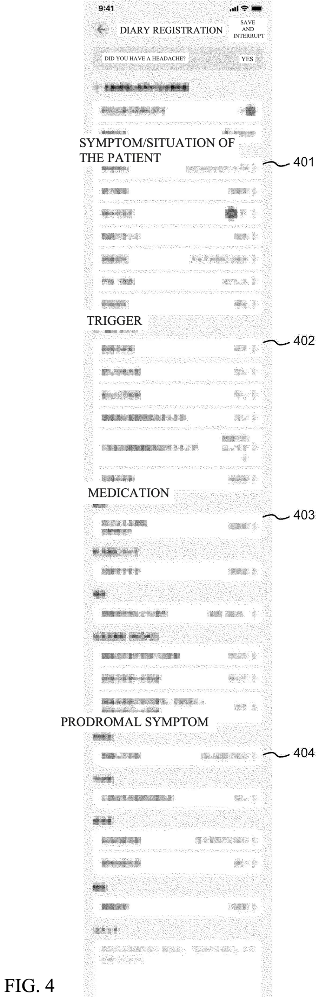

FIG. 4 is a detailed view of the "Diary Record" screen 22 (pop-up display from FIG. 3(b)).

In FIG. 4, reference numeral 401 denotes a field for inputting the "Patient's Symptom/Condition," 402 denotes a field for inputting "Triggers," 403 denotes a field for inputting "Medication," and 404 denotes a field for inputting "Prodromal symptom."

Most input items in the above fields are presented as pull-down menus from which the user selects an option. Furthermore, any of the options can be presented as preselected based on the information registered in the previous entry by the user, thereby reducing the burden of data entry for the user.

In the "Cognitive Restructuring" screen, information for promoting cognitive restructuring is provided to the user.

Specifically, the screen transitions sequentially according to the following steps, displaying information for promoting cognitive restructuring.

### <Step 1 - Select a Past Headache Episode>

From the most recent five headache episodes (headache scenes) registered in the headache diary, the user selects one episode that was particularly severe or memorable. The "headache scene" displays the headache symptoms, prodromal symptoms, triggers, and other relevant information.

When the "Next" button is selected by the user, the process proceeds to the next step (Step 2).

### <Step 2 - Recall the Headache Situation>

In Step 2, more detailed information is displayed regarding the headache episode selected in Step 1, including "Location" and "Stressful Events in Daily Life." This information is also based on the contents registered in the headache diary.

When the "Next" button is selected by the user, the process proceeds to the next step (Step 3).

### <Step 3 - Recall Thoughts>

In Step 3, the user is prompted to recall their "thoughts" during the headache episode selected in Step 1, by selecting from four levels for each item related to the "pain" of the headache.

When the "Next" button is selected by the user, the process proceeds to the next step (Step 4).

### <Step 4 - Recall Feelings>

In Step 4, the user recalls the "feelings" that came to his/her mind during the headache episode selected in Step 1. Specifically, multiple options representing "feelings" are presented, and the user is prompted to recall "feelings" by rating each on a four-point scale.

When the "Next" button is selected by the user, the process proceeds to the next step (Step 5).

### <Step 5 - Organize Thoughts>

In Step 5, the user acquires new ways of thinking about headaches during an attack by given questions and selection of options to the questions.

In acquiring the "new ways of thinking," three perspectives are provided:
1. "Focus on the evidence or reasons for the thought"
2. "Change the viewpoint"
3. "Notice cognitive distortions"

When the "Next" button is selected by the user, the process proceeds to the next step (Step 6).

### <Step 6 - Check Change in Feelings>

In Step 6, the user is prompted to reassess the headache episode selected in Step 1 using the newly acquired way of thinking from Step 5 and to consider what changes have occurred in their feelings.

Specifically, multiple options representing "feelings" are again presented, and the user is prompted to recall "feelings" by rating each on a four-point scale.

When the "Next" button is selected by the user, the process proceeds to the next step (Step 7).

### <Step 7 - Consider If There Are Newly Arising Feelings>

In Step 7, the user is prompted to reassess the headache episode selected in Step 1 using the newly acquired way of thinking from Step 5 and to consider if any new feelings have arisen.

Specifically, "Positive," "Relieved," and "Sense of Accomplishment" are displayed as the "Feelings" and the user is prompted to rate each on a four-point scale.

When the "Next" button is selected by the user, the process proceeds to the next step (Step 8).

### <Step 8 - Recall Results>

In Step 8, the results selected in Steps 1 through 7 are displayed, promoting the user to objectively confirm the changes in his/her way of thinking brought about through cognitive behavioral therapy.

The results of past sessions of cognitive behavioral therapy can also be viewed in a list

As described above, in Embodiment 1 of the present disclosure, when prompting the user to make cognitive restructuring, information for recalling situations in which a symptom (e.g., headache) occurred in the past is generated based on records in a symptom diary (e.g., headache diary) and provided to the user. Thus, the user can accurately recall past situations without requiring significant time or effort for retrospection, thereby alleviating difficulties and distress (including the time and effort required to recall the episode, the possibility of vague or distorted memory, etc.) associated with recalling the patient's state, and enabling the provision of appropriate cognitive behavioral therapy. Furthermore, an embodiment of the present disclosure may include, for example, a computer-implemented method for assessing the degree of disability caused by headache, comprising calculating a MIDAS score based on patient condition records (e.g., headache diary and initial interview records) and evaluating headache disability based on the calculated MIDAS score.

### (Second Embodiment)

A method for supporting cognitive behavioral therapy according to the second embodiment of the present disclosure comprises keeping record of a symptom or condition of a patient and at least one item of categorical information selected from a trigger, medication, a prodromal symptom, date and time, a treatment, influence, and a location by selecting one item of information from options.

As described above, in the "Diary Record" screen shown in Fig. 4, most input items are presented as pull-down menus in which they are selected from options.

That is, the method makes the user record daily symptoms or conditions of the user and a symptom diary (for example, a headache diary) relating to a trigger, medication, a prodromal symptom, and the like by selecting from options, thereby the user can continue recording the symptom diary (for example, a headache diary) without requiring time or effort, even on busy or tired days.

Furthermore, the user does not have to worry about what to write, which makes keeping a diary less burdensome and allows it to be continued over a long period of time.

Through this, it becomes possible to accurately recall past situations based on continuous recording of the symptom diary (for example, a headache diary), and cognitive behavioral therapy can thereby be provided more effectively.

### (Third Embodiment)

A method for supporting cognitive behavioral therapy according to the third embodiment of the present disclosure comprises keeping record of a symptom or condition of a patient and at least one item of categorical information selected from a trigger, medication, a prodromal symptom, date and time, a treatment, influence, and a location by selecting one item of information from options with any one of the options preselected.

As described above, the pull-down menu in the "diary record" screen shown in Fig. 4 is presented with one of the options preselected.

Therefore, the user only needs to perform a selection operation if the option to be selected is different from the already selected option, thereby allowing some of the selection operations to be omitted and providing convenience. Accordingly, according to this embodiment, cognitive behavioral therapy can be provided effectively while reducing the user's effort.

### (Fourth Embodiment)

A method for supporting cognitive behavioral therapy according to the fourth embodiment of the present disclosure comprises that the selection of a preselected option in keeping record of a symptom or condition of a patient and at least one item of categorical information selected from a trigger, medication, a prodromal symptom, date and time, a treatment, influence, and a location is performed based on the patient's previously recorded information.

As described above, the pull-down menu in the "diary record" screen shown in Fig. 4 is presented with one of the options preselected based on the contents previously registered by the user.

Therefore, when the option to be selected is the same as the already selected option, the user can record the symptom diary (for example, a headache diary) while saving time and effort.

On the other hand, when the option to be selected is different from the already selected option, the user can easily notice changes in circumstances or emotions since the previous time, and such changes are easily be retained in memory.

In other words, according to this embodiment, cognitive behavioral therapy can be provided even more effectively while saving the user's time and effort.

### (Fifth Embodiment)

A method for supporting cognitive behavioral therapy according to the fifth embodiment of the present disclosure comprises keeping record of a symptom or condition of a patient by text input into a text field for a certain period, displaying a text candidate predicted from a few input letters, and performing the prediction of the text candidate based on the patient's previously recorded information.

The "text candidate" is a function that predicts and displays, as an input candidate, the words that are expected to be input into a text field or the like from the first few characters entered, and is common in the field of IT (Information Technology). The candidate text is displayed in order of frequency of occurrence of words, for example, on a network.

In the fifth embodiment of the present disclosure, when a text field is displayed and the patient is made to enter text in the record of a symptom diary (for example, a headache diary) that is the basis of information for recalling situations in which symptoms or conditions (for example, headache) occurred at past, text candidates are displayed based on information previously entered by the patient before that day. Therefore, even for free-text input fields, the information can be entered in accordance with the patient's situation or emotions at that time without requiring time or effort for input. Accordingly, it becomes possible to provide cognitive behavioral therapy effectively while alleviating the difficulty or burden associated with symptom recall (including, for example, the time and effort required to recall the episode of onset, the possibility of only vague recall of the episode, or distortion of memory).

### (Sixth Embodiment)

A method for supporting cognitive behavioral therapy according to the sixth embodiment of the present disclosure comprises performing statistical processing and/or clustering processing on data in a database in which a symptom or condition of multiple patients and at least one item of categorical information selected from a trigger, medication, a prodromal symptom, date and time, a treatment, influence, and a location are recorded for each of multiple patients, to create a plurality of patient classifications, and identifying a patient classification to which the patient belongs based on the symptom or condition of a patient recorded for a certain period and at least one item of categorical information selected from a trigger, medication, a prodromal symptom, date and time, a treatment, influence, and a location.

The term "clustering" is a type of machine learning and refers to a method of grouping data based on similarity of the data.

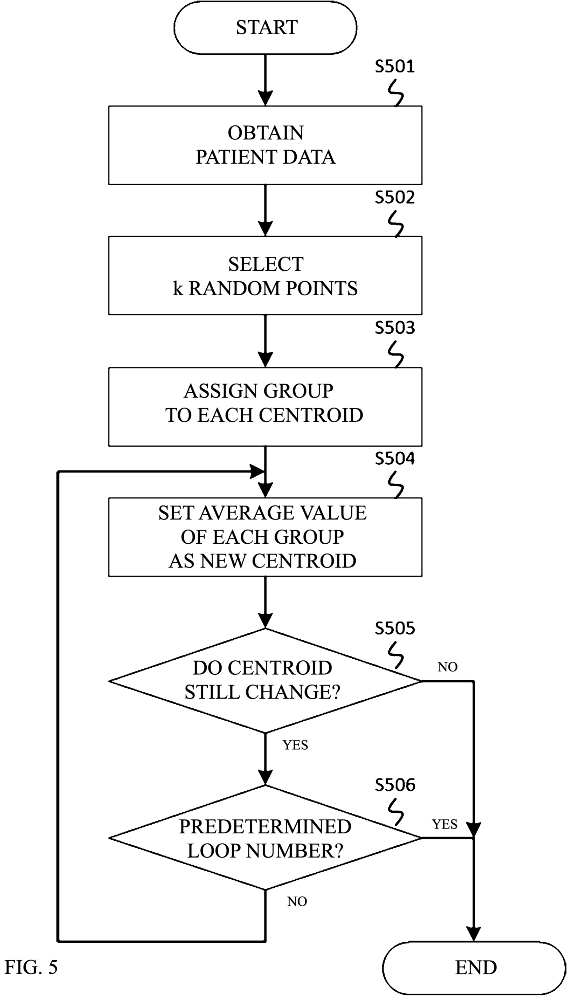

Fig. 5 shows, as an example of statistical processing or clustering processing, a flow (flow chart) of processing for creating patient classifications by performing k-means clustering, which is a type of non-hierarchical clustering, on data in the database of a plurality of patients. In this example, headache patients are used as an example.

First, data of headache patients is acquired from the database (S501). In the data diary records consisting of symptoms or conditions of a patient and at least one of a trigger, medication, a prodromal symptom, date and time, a treatment, influence, and a location are recorded in association with information such as patient ID, age, sex, preexisting conditions, occupation/working hours, and family structure.

Next, k random points (called centroids) are selected (S502). Here, k is a natural number indicating the number of groups for classification.

Next, the centroid nearest each data are found, and the data is assigned to the group belonging to the centroid (S503).

Next, the average value of the data in each group is calculated and set as a new centroid (S504).

The centroid determines the change (S505), and when the centroid do not change any more (YES in S505), the processing ends.

The loop number is also determined (S506), and when a predetermined number is reached (YES in S506), the processing ends.

By analyzing the characteristics and trends of each group thus obtained, patient classifications can be created.

Then, for a given patient, it becomes possible to predict changes in symptoms or conditions when treatment (treatment by cognitive behavioral therapy) is carried out, based on the data of other patients belonging to the same group and preceding in the therapy period or phase.

It becomes also possible to provide an appropriate therapeutic approach (an appropriate combination of questions and options) based on the data of other patients belonging to the same group who are showing favorable therapeutic effects.

For example, by displaying the text previously entered by other patients showing favorable therapeutic effects at the top of the text conversion candidate list in the free text input field, it is expected that similar favorable cognitive restructuring will be promoted.

That is, the text displayed as the text conversion candidate is the text entered by other patients belonging to the same group. For example, in the case of a user belonging to a younger age group, so-called "youth slang" is displayed, so that the user can input the text as their own words without discomfort and find it easier to sympathize with the contents.

As described above, according to this embodiment, cognitive behavioral therapy can be provided even more effectively in accordance with the characteristics and tendencies of patients.

### (Seventh Embodiment)

A method for supporting cognitive behavioral therapy according to the seventh embodiment of the present disclosure comprises presenting information on the relationship between a symptom or condition of a patient recorded for a certain period and the frequency of occurrence of at least one item of information selected from a trigger, medication, a prodromal symptom, date and time, a treatment, influence, and a location to the patient. An embodiment according to the present disclosure may be, for example, a computer-implemented method for supporting cognitive behavioral therapy, comprising keeping record of a symptom or condition and at least one item of categorical information selected from a trigger, medication, and a prodromal symptom for a certain period, and presenting information on the relationship between the symptom or condition of a patient recorded for a certain period and the frequency of occurrence of at least one item of information selected from a trigger, medication, and a prodromal symptom to the patient.

By presenting the relationship between symptoms or conditions of a patient in a symptom diary (for example, a headache diary) and items such as "trigger," "medication," and "prodromal symptom," the following advantages can be obtained.

First, it becomes possible to understand occurrence patterns and characteristics of symptom (for example, headache). Examples include the degree or duration of the symptom (for example, headache), the time of day or day of the week when the symptom occurs, and associated symptoms or prodromal symptoms.

Further, triggers of the patient's symptom (for example, headache) can be identified. Examples include diet, alcohol consumption, sleep, stress, temperature, and atmospheric pressure.

It also becomes possible to evaluate the patient's medication status and effectiveness, such as timing and frequency of drug administration, presence or absence and duration of effects, and presence or degree of side effects.

As described above, according to this embodiment, it becomes possible to deepen the understanding of the patient's symptoms and to provide cognitive behavioral therapy more appropriately.

### (Eighth Embodiment)

A method for supporting cognitive behavioral therapy according to an eighth embodiment of the present disclosure comprises displaying symptoms or conditions of the patient recorded for a certain period and the frequency of occurrence of at least one item of information selected from a trigger, medication, a prodromal symptom, date and time, a treatment, influence, and a location in a ranked order.

By displaying the symptoms or conditions of a patient in a symptom diary (for example, a headache diary) and the frequency of occurrence of "trigger," "medication," and "prodromal symptom" in a ranked order, the following advantages can be obtained.

First, it becomes possible to preferentially understand important factors or influences on the patient's symptom (for example, headache). For example, triggers most closely related to the occurrence of the symptom (for example, headache) or the medication most effective in reducing the symptom (for example, headache).

Furthermore, it becomes possible to set appropriate treatment plans and goals for the patient. Examples include what preventive measures or treatments are necessary to most strongly suppress symptom (for example, headache) occurrence and how much improvement is desirable in symptom (for example, headache) intensity or duration.

In addition, it is possible to evaluate therapeutic effects and progress of the patient. Examples include how much the frequency or severity of symptom (for example, headache) occurrence has decreased, or how much the frequency of occurrence of triggers, medication, and prodromal symptoms has changed.

As described above, according to this embodiment, it becomes possible to make the importance and priority of the patient's symptom (for example, headache) clear and provide cognitive behavioral therapy that enables efficient treatment.

### (Ninth Embodiment)

A method for supporting cognitive behavioral therapy according to the ninth embodiment of the present disclosure comprises providing a patient with information that promoting cognitive restructuring and a video explaining the content of cognitive restructuring.

Here, the term "promote" should be interpreted in the general sense of "to advance or enhance" and should not be construed as conditional instructions such as "to implement when the categorical information is A but not to implement when it is B."

In recent years, watching short videos of a few minutes on mobile devices such as smartphones has become widespread, particularly among younger generations. Providing videos explaining the content of cognitive restructuring matches such viewing habits and is expected to further promote cognitive restructuring. In other words, merely watching a video explaining the content of cognitive restructuring can be expected to produce psychological effects equivalent to performing cognitive restructuring.

Accordingly, according to this ninth embodiment of the present disclosure, behavioral activation can be provided more appropriately.

### (Tenth Embodiment)

A method for supporting cognitive behavioral therapy according to a tenth embodiment of the present disclosure comprises providing a patient with information that promotes behavioral activation and a video explaining the content of behavioral activation.

Behavioral activation refers to increasing meaningful activities in line with one's own sense of values or enjoyable activities and is based on the theory that psychological problems are exacerbated by reduced or avoidant activities.

For example, headache is often affected by lifestyle habits such as stress, tension, and lack of sleep. When headache occurs, activities may be further restricted, leading to a vicious cycle. Behavioral activation is expected to have effects such as preventing headache attacks and alleviating the pain associated with attacks.

Providing, together with information that promotes behavioral activation, a video explaining the content of behavioral activation matches the aforementioned habit of watching short videos on information terminals, and is expected to further promote behavioral activation. In other words, merely watching a video explaining the content of behavioral activation can be expected to produce psychological effects equivalent to actually performing the behavior.

Accordingly, according to this tenth embodiment of the present disclosure, behavioral activation can be provided more appropriately.

### (Eleventh Embodiment)

A method for supporting cognitive behavioral therapy according to the eleventh embodiment of the present disclosure comprises providing a patient with information that promote relaxation and a video explaining the content of relaxation.

In the application program according to the present disclosure, "relaxation methods," such as "breathing techniques" and "muscle relaxation methods," which allow the user to relax at the time of symptom onset (for example, headache attack), are provided to the users to reduce physical tension and discomfort.

Providing, together with information that promotes relaxation, a video explaining the content of relaxation matches the aforementioned habit of watching short videos on information terminals, and is expected to further promote relaxation. In other words, merely watching a video explaining the content of relaxation can be expected to produce psychological effects such as feeling relaxed.

Accordingly, according to this eleventh embodiment of the present disclosure, cognitive behavioral therapy can be provided more appropriately.

### (Twelfth Embodiment)

A method for supporting cognitive behavioral therapy according to the twelfth embodiment of the present disclosure comprises providing a patient with information that promotes biofeedback and a video explaining the content of the biofeedback.

Biofeedback refers to a training method in which biological signals or physiological information are measured and it is fed back to the patient or user in real time to promote self-awareness and self-regulation. The biological signals measured in biofeedback include heart rate, skin electrical activity (galvanic skin response), muscle tension, brain waves, and the like.

The basic mechanism of the biofeedback proceeds as follows.

First, biological signals are measured using sensors or devices. The measured data are analyzed by a computer, and the analysis results are presented to the patient or user in real time. Presentation methods include visual feedback such as graphs or numerical values, auditory feedback such as voice or sound, and tactile feedback such as vibration.

Biofeedback is used for stress management, treatment of anxiety disorders, pain reduction, improvement of physical performance, and improvement of sleep.

For example, in "heart rate biofeedback," heart rate is measured using an electrocardiogram sensor, and the heart rate is presented to the patient in real time. When the patient's heart rate is high, the patient attempts methods such as relaxation techniques or deep breathing to lower the heart rate.

In "muscle biofeedback," muscle tension is measured using an electromyogram sensor and presented to the patient in real time. The patient becomes aware of areas of high tension and aims to relax the muscles using relaxation techniques or muscle relaxation methods.

In "brain wave biofeedback," brain waves are measured using a brain wave sensor and brain wave patterns are presented to the patient in real time. The patient trains to increase or decrease specific brain wave patterns and aims to adjust the state of the brain. Brain wave biofeedback is sometimes used for attention enhancement or concentration training.

Providing, together with information that promotes biofeedback, a video explaining the content of biofeedback matches the aforementioned habit of watching short videos on information terminals, and is expected to further promote biofeedback.

Accordingly, according to this twelfth embodiment of the present disclosure, cognitive behavioral therapy can be provided even more appropriately.

### (Thirteenth Embodiment)

A method for supporting cognitive behavioral therapy according to the thirteenth embodiment of the present disclosure further comprises keeping record of the state of a patient after implementation of cognitive behavioral therapy and comparing the patient's state before implementation of cognitive behavioral therapy with the state after implementation of cognitive behavioral therapy. An embodiment according to the present disclosure may include, for example, a computer-implemented method for supporting cognitive restructuring, comprising keeping record of a first state of the patient as a baseline, providing the patient with information for recalling situations in which symptoms (for example, headache) occurred in the past, providing the patient with information that promote cognitive restructuring, keeping record of a second state of the patient after cognitive restructuring, and comparing the first state of the patient with the second state of the patient.

In the method for supporting cognitive behavioral therapy according to the present disclosure, by providing the patient with information comparing the patient's state before and after implementation of cognitive behavioral therapy, the "visualization" of cognitive changes is achieved. In other words, the patient can intuitively understand the effect of cognitive behavioral therapy. That is, when symptoms are alleviated or the frequency or intensity of symptoms (for example, headache) decreases, the patient can become aware of the influence that changes in their cognition or thinking exert on the symptoms (for example, headache).

Further, by comparing the state before implementation with the state after implementation, self-efficacy and motivation can be improved. By seeing the results of symptom improvement, the patient can realize that their treatment efforts and cognitive changes are actually producing results, which increases their willingness to continue their efforts.

In addition, by comparing the state before implementation with the state after implementation, self-observation is promoted. The patient can objectively understand the patterns and triggers of their symptoms (for example, headache) and the characteristics of their cognition, thereby it becomes possible to learn how to manage and prevent future symptoms.

Furthermore, by comparing the state before implementation with the state after implementation, it becomes possible to identify and correct cognitive problems and distorted thinking patterns. Recalling their thoughts and beliefs at the time when symptoms (for example, headache) occurred and finding healthier and more constructive approaches lead to the prevention or alleviation of future symptoms.

In summary, by comparing the state before and after implementation of cognitive behavioral therapy, it becomes possible to visualize treatment progress for the patient, enhance treatment motivation, promote self-observation and learning, and support cognitive correction and progress. This makes it possible to provide cognitive behavioral therapy even more appropriately. An embodiment according to the present disclosure may include, for example, a computer-implemented method for generating a learning curriculum, comprising selecting learning content to be included in the learning curriculum based on patient information, wherein the learning content is tagged with one or more pieces of first keyword information representing the information of the learning content, and the patient information includes one or more pieces of second keyword information derived from patient attributes, interview results, patient condition records, or measurement results of patient biological information obtained by a measurement device, and the selection of learning content is made based on at least one match between the first keyword information and the second keyword information.

### (Fourteenth Embodiment)

A method for supporting cognitive behavioral therapy according to the fourteenth embodiment of the present disclosure further comprises registering multiple scheduled dates and times for training of cognitive behavioral therapy for a certain period, providing a patient with an explanatory video when the patient performs training, keeping record of whether the training was performed at each scheduled date and time, keeping record of the patient's impressions after the training when the training is performed, and presenting information regarding the relationship between the training implementation status and impressions and the symptoms or conditions of the patient recorded for a certain period, to the patient. An embodiment according to the present disclosure may include, for example, a computer-implemented method for supporting cognitive behavioral therapy, comprising keeping record of the symptoms or conditions of the patient for a certain period, registering multiple scheduled dates and times for training of cognitive behavioral therapy for a certain period, optionally prompting the patient to perform training a predetermined period before the scheduled training date and time, providing an explanatory video when the patient performs training, keeping record of whether the training was performed at each scheduled date and time, and keeping record of the patient's impressions after the training when the training has been performed, characterized by presenting information regarding the relationship between the recorded training implementation status and impressions and the symptoms or conditions of the patient for a certain period, to the patient.

In the method for supporting cognitive behavioral therapy according to the present disclosure, registering scheduled training dates and times, keeping record of whether the training was performed and the impressions, and associating that information with symptoms (for example, headache symptoms) provides the following advantages.

By tracking the relationship between training implementation status and symptoms (for example, headache symptoms), the effect of cognitive behavioral therapy can be objectively evaluated. For example, if the frequency or intensity of symptoms (for example, headache) decreases after training is performed, it is highly possible that the training contributes to the improvement of symptoms (for example, headache).

By keeping record of the training implementation status and symptoms (for example, headache symptoms) and comparing them, it becomes possible to identify patterns and triggers of symptoms (for example, headache). If a specific training is effective in alleviating symptoms (for example, headache), it may be suggested by recording it that certain situations or thought patterns are involved in the symptoms (for example, headache), thereby helping to identify appropriate countermeasures.

By keeping record of training implementation status and impressions, users themselves can actively engage in their own treatment process and deepen self-observation. Clarifying the relationship between symptoms (for example, headache) and training can encourage the users to face their own symptoms and enhance their willingness to continue their treatment plans.

By analyzing the recorded training implementation status and impressions, it is possible to obtain clues for creating an optimal treatment plan for each user. By identifying approaches or training which are particularly effective for symptoms (for example, headache symptoms) and providing them to the user, treatment outcomes can be maximized.

In summary, it is possible to support the construction of effective treatment strategies and management of the user's symptoms (for example, headache symptoms), including evaluation of treatment effects, identification of patterns, promotion of user engagement, and improvement of individualized treatment plans. This becomes possible to provide cognitive behavioral therapy more appropriately.

### (Fifteenth Embodiment)

A method for supporting cognitive behavioral therapy according to the fifteenth embodiment of the present disclosure further comprises prompting the patient to perform the training a predetermined period before the scheduled training date and time.

In the method for supporting cognitive behavioral therapy according to the present disclosure, prompting the patient to perform the training a predetermined period before the scheduled training date and time has the following advantages.

By prompting the training before the scheduled date and time, it is possible to enhance the patient's motivation. That is, reminding the patient that the scheduled date and time is approaching serves as a trigger to actively engage in the training.

In addition, prompting the training a predetermined period before the scheduled date and time enables the patient to be reminded of continuing the regular training. It becomes possible that the training is habitual and the treatment process progresses continuously rather than intermittently.

Furthermore, by prompting training a predetermined period before the scheduled date and time, it becomes possible to adjust and support the treatment plan. If there are reasons or obstacles preventing implementation at the scheduled date and time, they can be addressed earlier. In other words, by providing necessary resources or support, smooth implementation of the training can be achieved.

Moreover, prompting training a predetermined period before the scheduled date and time enables tracking and evaluating the patient's training performance. By understanding whether the training was implemented and/or changes in symptoms (for example, headache symptoms), it is possible to monitor the treatment progress and adjust the treatment plan if necessary.

In some embodiments, the method according to the present disclosure further comprises prompting the patient so that the number of days on which records are made during a certain period is, for example, 40% or more, 50% or more, 60% or more, 70% or more, 80% or more, 90% or more, or 95% or more. The application recording rate of migraine is defined as the ratio (%) of the number of days on which symptom records are made during the trial period [application recording rate of migraine (%) = (number of days on which condition records were made / actual number of days of the trial period) × 100].

In summary, by prompting the patient to perform training a predetermined period before the scheduled training date and time, it is possible to enhance patient motivation, ensure continuity of training, adjust and support the treatment plan, and track and evaluate treatment outcomes. This enables cognitive behavioral therapy to be provided even more effectively.

### (Other Embodiments)

The present invention is not limited to the above embodiments, and various changes and modifications can be made without departing from the spirit and scope of the invention.

Furthermore, the present invention may also be realized by supplying software (programs) for implementing the functions of the above embodiments to a system or apparatus via a network or various storage media, and causing a computer (or CPU, MPU, or the like) of the system or apparatus to read and execute the program. In some embodiments, the device according to the present disclosure may include a processor for performing various processes and a memory connected to the processor. In some embodiments, the device according to the present disclosure may be realized by executing the program of the present disclosure on a general-purpose computer. In some embodiments, the apparatus according to the present disclosure comprises a storage device storing a computer program of the present disclosure and a processor capable of executing instructions of the program. The present invention also relates to a non-transitory computer-readable recording medium storing the program of the present disclosure. Examples of computer-readable recording media include, but are not limited to, hard disk drives (HDD), solid-state drives (SSD), flash memory (such as USB memory and SD cards), optical disks (such as CDs, DVDs, and Blu-ray disks), magnetic tapes, floppy disks, and cloud storage.

Accordingly, the program code itself to be installed on a computer to realize the functional processing of the present invention with the computer realize the present invention. That is, the present invention includes the computer program itself for realizing the functional processing of the present invention. In this case, it may take the form of object code, a program executed by an interpreter, a script or macro executed by an application program such as a web browser, or an API (Application Programming Interface) as long as it has function of a program. Furthermore, it may be integrated and implemented as a part of another web service such as a Social Networking Service (SNS) using web programming techniques such as "mashup."

In addition, the present invention can also be implemented as a so-called web application using a web browser. Therefore, the web server indicated by the URL (Uniform Resource Locator) constitutes the implementation of the present invention, regardless of whether it is a single hardware or not.

The embodiments described in this specification are intended to be merely illustrative, and those skilled in the art will be able to make numerous modifications and changes without departing from the spirit of the invention. Some modifications and changes may not achieve optimal results, but may still yield satisfactory outcomes. All such modifications and changes are intended to fall within the scope of the invention as defined by the appended claims. Any combination of the elements disclosed herein, and any transformations of the present disclosure's descriptions between methods, devices, systems, computer programs, data structures, recording media, and so on, are also valid embodiments of the present disclosure. Accordingly, the details described for the method of the present disclosure may be applied to systems, computer programs, data structures, recording media, and the like.

The present disclosure will be further understood with reference to the following examples. These examples are provided solely for illustrating the disclosure described in the claims, and the present disclosure is not limited to the illustrated embodiments but is intended merely as examples of embodiments of the present disclosure. Any functionally equivalent methods are included within the scope of the present disclosure. In addition to what has been described herein, various modifications of the present disclosure will be apparent to those skilled in the art from the above description. Such modifications are intended to fall within the scope of the appended claims.

### [Examples]

### Example 1 (Clinical Study)

### (1) Study Design

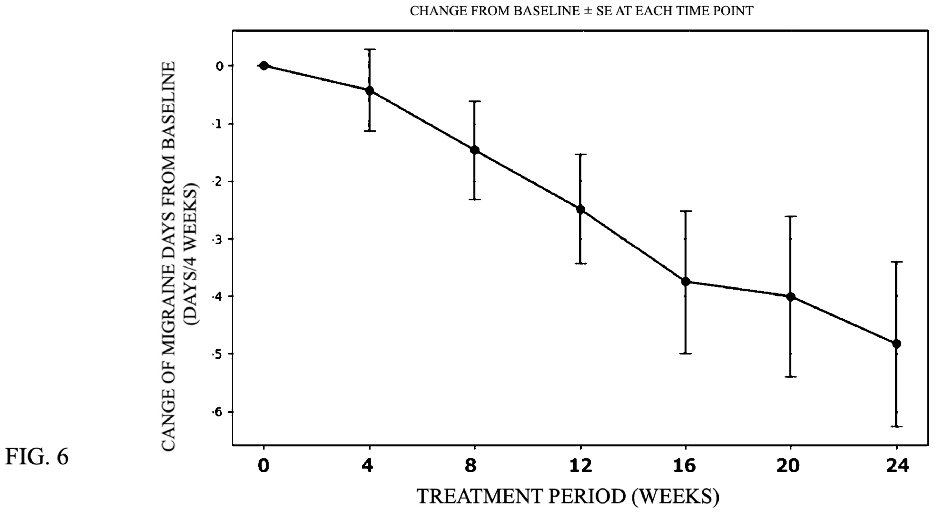

Fifty-two patients who experienced migraine attacks at least four days per month were treated using this headache treatment application for 24 weeks. During this period, patients recorded their symptoms or conditions and at least one item of categorical information selected from triggers, presence/absence and/or detail of medication, prodromal symptoms, date and time, treatments, influence, and locations. Based on these records, information for recalling situations in which symptoms occurred in the past, information for learning about their own symptoms or conditions (e.g., headache), and information promoting cognitive behavioral therapy were presented to the patients. Changes in the number of migraine days per 4 weeks from baseline at 4, 8, 12, 16, 20, and 24 weeks of treatment are shown in Figure 6.

### (2) Results

The results are shown in Figure 6. Compared with the "number of migraine days per 4 weeks (baseline value)" before treatment with this headache treatment application, the "number of migraine days per 4 weeks" decreased over time up to 24 weeks of treatment with the headache treatment application. That is, treatment with the headache treatment application, which has both the "function for learning about one's own headache" and the "function for promoting cognitive behavioral therapy," markedly suppressed migraine attacks and demonstrated high efficacy. No adverse events were observed.

### Example 2 (Clinical Study)

### (1) Study Design

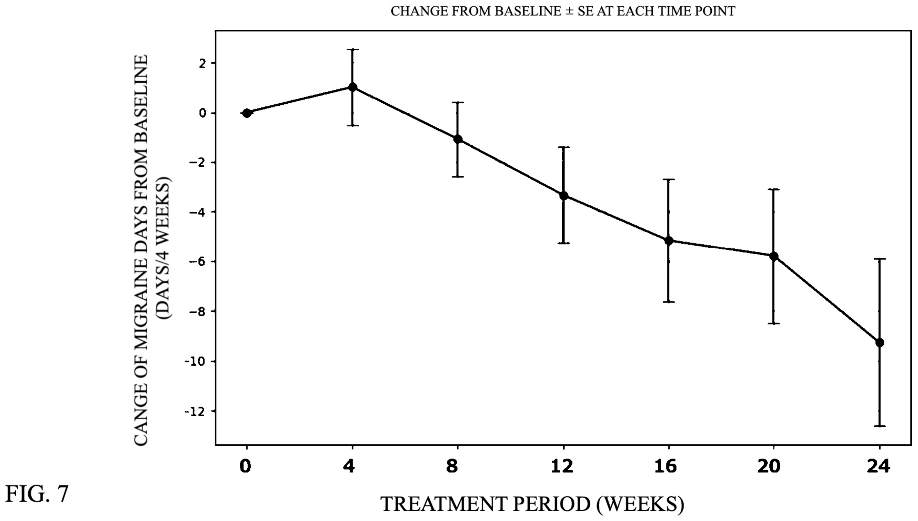

In 2021, antibodies against calcitonin gene-related peptide (CGRP) and antibodies against the CGRP receptor were approved in Japan as therapeutic drugs for migraine, making them available for patients with insufficient response to conventional migraine treatment. However, some patients still exhibit insufficient response even to treatment with the anti-CGRP antibodies or the anti-CGRP receptor antibody. Therefore, the effect of this headache treatment application was examined in 12 patients who experienced migraine attacks at least four days per month and had insufficient response to treatment with the anti-CGRP antibodies or the anti-CGRP receptor antibody. These patients were treated with the headache treatment application for 24 weeks. Changes in the number of migraine days per 4 weeks from baseline at 4, 8, 12, 16, 20, and 24 weeks of treatment are shown in Figure 7.

### (2) Results

The results are shown in Figure 7. In patients with insufficient response to treatment with the anti-CGRP antibodies or the anti-CGRP receptor antibody, compared with the "number of migraine days per 4 weeks (baseline value)" before treatment with the headache treatment application, the "number of migraine days per 4 weeks" decreased over time up to 24 weeks of treatment. That is, treatment with the headache treatment application, which has both the "function for learning about one's own headache" and the "function for promoting cognitive behavioral therapy," markedly suppressed migraine attacks and demonstrated high efficacy even in patients with insufficient response to treatment with anti-CGRP antibodies or anti-CGRP receptor antibodies. No adverse events were observed.

### [Explanation of Reference Numerals]

- 100: Computer
- 101: Control unit
- 102: Storage unit
- 103: Peripheral device I/F unit
- 104: Input unit
- 105: Display unit
- 106: Communication unit
- 110: Bus
- 120: Network
- 130: External server
- 140: Database
- 21: Home screen
- 22: Diary record screen
- 23: My page screen
- 24: Learning screen
- 25: Analysis screen
- 26: Training screen

## Claims

1. A computer-implemented method for supporting cognitive behavioral therapy, the method comprising:
keeping record of a symptom or condition of a patient and at least one item of categorical information selected from a trigger, presence/absence and/or detail of medication, a prodromal symptom, date and time, a treatment, influence, and a location for a certain period;
generating and providing the patient with information for recalling a past situation in which the symptom occurred, based on the record of the symptom or condition and the categorical information;
providing the patient with information for learning about the symptom or condition of the patient; and
providing the patient with information that promotes the cognitive behavioral therapy.

2. The method according to claim 1, further comprising keeping record of the symptom or condition and the categorical information by selecting one item of information from options thereof

3. The method according to claim 2, wherein the options in the record of the symptom or condition and the categorical information are provided with one of the options preselected.

4. The method according to claim 3, wherein the selection of the preselected option in the record of the symptom or condition and the categorical information is made based on a record of past information of the patient.

5. The method for supporting cognitive behavioral therapy according to any one of claims 1 to 4, further comprising:
keeping record of a symptom or condition of a patient by text input into a text field for a certain period; and
displaying a text candidate predicted from a few input letters; the text candidate being predicted based on past records of information of the patient.

6. The method for supporting cognitive behavioral therapy according to any one of claims 1 to 5, further comprising:
performing statistical processing and/or clustering processing on data in a database made by keeping record of symptoms or conditions of multiple patients and at least one item of categorical information selected from a trigger, presence/absence and/or detail of medication, a prodromal symptom, date and time, a treatment, influence, and a location for a certain period to create multiple patient classifications; and
identifying a patient classification to which a patient belongs, based on a symptom or condition of the patient recorded for a certain period and the categorical information selected from a trigger, presence/absence and/or detail of medication, a prodromal symptom, date and time, a treatment, influence, and a location.

7. The method for supporting cognitive behavioral therapy according to any one of claims 1 to 6, further comprising:
presenting, to the patient, information on the relationship between a symptom or condition of a patient recorded for a certain period and frequency of occurrence of at least one item of information selected from a trigger, presence/absence and/or detail of medication, effects of medication, a prodromal symptom, date and time, a treatment, influence, and a location.

8. The method for supporting cognitive behavioral therapy according to any one of claims 1 to 7, further comprising r displaying a symptom or condition of a patient recorded for a certain period and frequency of occurrence of at least one item of information selected from a trigger, presence/absence and/or detail of medication, effects of medication, a prodromal symptom, date and time, a treatment, influence, and a location in a ranked order.

9. The method for supporting cognitive behavioral therapy according to any one of claims 1 to 8, further comprising providing the patient with information promoting cognitive restructuring based on the symptom or condition and the categorical information and/or a video explaining content of cognitive restructuring.

10. The method for supporting cognitive behavioral therapy according to any one of claims 1 to 9, further comprising providing the patient with information promoting behavioral activation based on the symptom or condition and the categorical information and/or a video explaining content of behavioral activation.

11. The method for supporting cognitive behavioral therapy according to any one of claims 1 to 10, further comprising providing the patient with information promoting relaxation based on the symptom or condition and the categorical information and/or a video explaining content of relaxation.

12. The method for supporting cognitive behavioral therapy according to any one of claims 1 to 11, further comprising providing the patient with information promoting biofeedback based on the symptom or condition and the categorical information and/or a video explaining contentof biofeedback.

13. The method for supporting cognitive behavioral therapy according to any one of claims 1 to 12, further comprising:
keeping record of the patient's condition after the implementation of cognitive behavioral therapy; and
comparing the patient's condition before and after the implementation of cognitive behavioral therapy.

14. The method for supporting cognitive behavioral therapy according to any one of claims 1 to 13, further comprising:
registering multiple dates/times scheduled for training of cognitive behavioral therapy for a certain period;
providing an explanatory video when the patient performs the training;
keeping record of whether the training was performed at each scheduled date and time;
when the training was conducted, keeping record of the patient's impressions after the training; and
presenting, to the patient, relationship between the symptom or condition of the patient and the training implementation status and impressions recorded for a certain period.

15. The method for supporting cognitive behavioral therapy according to claim 14, further comprising prompting the patient to perform the training a predetermined period before the scheduled training date and time.

16. A cognitive behavioral therapy support apparatus comprising:
a patient state recording means for keeping record of a symptom or condition of a patient and at least one item of categorical information selected from a trigger, presence/absence and/or detail of medication, a prodromal symptom, date and time, a treatment, influence, and a location for a certain period;
a recall information providing means for generating, based on the record of the symptom or condition and the categorical information, information for recalling a past situation in which the symptom occurred, and providing the patient with the information;
a learning information providing means for providing the patient with information for learning about the symptom or condition of the patient; and
a cognitive behavioral therapy information providing means for providing the patient with information promoting cognitive behavioral therapy.

17. A program for causing a computer to function as each means of the cognitive behavioral therapy support apparatus according to claim 16.

18. A computer-readable recording medium storing a program for causing a computer to function as each means of the cognitive behavioral therapy support apparatus according to claim 16.

19. The method for supporting cognitive behavioral therapy according to any one of claims 1 to 14, wherein the symptom or condition is migraine.

20. The method for supporting cognitive behavioral therapy according to claim 19, wherein the patient is a patient who has shown insufficient response to treatment with an anti-CGRP antibody or an anti-CGRP receptor antibody.

21. The method for supporting cognitive behavioral therapy according to any one of claims 1 to 14, wherein the symptom or condition is PMS.

22. The method for supporting cognitive behavioral therapy according to any one of claims 1 to 14, further comprising prompting the patient so that the number of days on which the record is kept accounts for 40% or more of the days in a certain period.

23. The method for supporting cognitive behavioral therapy according to any one of claims 1 to 14, wherein the symptom or condition is selected from the group consisting of a headache, a tension-type headache, a medication overuse headache, a hormonal headache, chronic low back pain, chronic pain syndrome (CRPS), fibromyalgia, joint pain, PMDD (premenstrual dysphoric disorder), menstrual-related disorder, depression, anxiety disorder, panic disorder, social anxiety disorder, post-traumatic stress disorder, obsessive-compulsive disorder, insomnia, eating disorder, schizophrenia, and mental disorder.

24. The method for supporting cognitive behavioral therapy according to any one of claims 1 to 14, wherein the information for recalling a past situation in which a symptom occurred includes at least one selected from the group consisting of headache symptom, a prodromal symptom, a concomitant symptom, a trigger, a location at the time of attack, level of physical fatigue, level of mental fatigue, and sleep duration.

25. The method for supporting cognitive behavioral therapy according to claim 24, wherein the information for recalling a past situation in which a symptom occurred comprises at least a prodromal symptom and a concomitant symptom.
